# FASCICULE DE BREVET EUROPEEN

(11) **EP 2 378 968 B1**
(45) Date de publication et mention de la délivrance du brevet: **20.04.2016**
(21) Numéro de dépôt: 09755901.7
(22) Date de dépôt: 18.11.2009
(51) Int. Cl.: A61B 5/103

(54) **DISPOSITIF ET PROCEDE DE PREVENTION DE LA FORMATION D'ESCARRES**
VORRICHTUNG UND VERFAHREN ZUR PRÄVENTION DES AUFTRETENS VON SCHORF
DEVICE AND METHOD FOR PREVENTING THE OCCURRENCE OF ESCHARS

(30) Priorité: 21.11.2008 FR 0857907
(43) Date de publication de la demande: 26.10.2011
(73) Titulaire: Université Grenoble Alpes, 38400 Saint-Martin-d'Hères (FR); Centre National de la Recherche Scientifique (CNRS), 75016 Paris (FR); Informatique de Sécurité, 71300 Montceau-les-Mines (FR)
(72) Inventeur: VUILLERME, Nicolas, F-73800 Francin (FR); PAYAN, Yohan, F-38580 Allevard (FR); CHENU, Olivier, F-38000 Grenoble (FR); DIOT, Bruno, F-71640 Mercurey (FR); CANNARD, Francis, F-21200 Beaune (FR); LAVARENNE, Christophe, F-71100 St Remy (FR)
(74) Mandataire: Regimbeau
(86) Numéro de dépôt international: PCT/EP2009/065403
(87) Numéro de publication internationale: WO 2010/057926

(56) Documents cités:
- WO-A-01/00089
- FR-A- 2 872 030
- US-A- 6 030 351
- US-A1- 2004 054 303
- US-A1- 2007 182 570

## Description

### DOMAINE DE L'INVENTION

La présente invention concerne un dispositif et un procédé de prédiction et de prévention de la formation d'escarres chez une personne, notamment en vue de permettre le maintien à domicile de personnes paraplégiques et/ou âgées.

### ARRIERE PLAN DE L'INVENTION

La prévention des escarres chez des personnes immobilisés en position assise ou couchée est un problème important dans le milieu médical, notamment lorsque les personnes ne sont pas hospitalisées mais à leur domicile.

Des dispositifs permettant de surveiller le risque d'apparition des escarres, et d'alerter les personnes, ont donc été développés.

Ainsi, le document WO 2006/008406 décrit un fauteuil dont le siège est équipé d'une matrice de capteurs de pression permettant de mesurer la pression au niveau des zones d'appui de la personne assise. Des mesures de pression sont effectuées à intervalles réguliers, par exemple toutes les secondes, et comparées à un seuil prédéterminé. Une alerte de risque d'apparition d'escarres est déclenchée lorsqu'une pression mesurée est supérieure à un seuil prédéterminé pendant une certaine durée, ledit seuil étant défini indépendamment des caractéristiques et habitudes posturales de la personne. Le dispositif de mesure est associé à un dispositif d'électro-stimulation linguale permettant d'indiquer à la personne comment modifier sa position pour éviter les surpressions fessières prolongées et ainsi l'apparition d'escarres.

Or, chaque personne, en fonction de ses caractéristiques morphologiques (indice de masse corporelle,...), neurophysiologiques, de son activité, ... présente un niveau de risque qui lui est propre.

Il en résulte que, le seuil d'alerte fixé étant faible (pour prévenir l'apparition des escarres chez les personnes les plus à risques), l'alerte se déclenche inutilement chez les personnes moins à risques.

On cherche donc à minimiser l'intrusion du système d'alerte dans la vie de la personne, et à optimiser son déclenchement pour que chaque personne ne soit alertée que lorsqu'un risque réel existe.

Par ailleurs, les dispositifs actuels enregistrent des signaux en continu.

Or, toutes les situations ne donnent pas nécessairement lieu à la formation d'escarres.

Il serait donc souhaitable de limiter les mesures aux périodes où la personne est la plus sujette à la formation d'escarres.

L'invention vise à proposer un dispositif de prévention des escarres qui favorise le maintien à domicile des personnes sujettes aux escarres et propose un seuil d'alerte personnalisé et évolutif.

Il doit en outre autoriser une surveillance à distance de la personne.

Par ailleurs, ce dispositif doit être le moins intrusif possible dans la vie de la personne, et présenter par conséquent une précision améliorée par rapport aux techniques de l'art antérieur.

Enfin, ce dispositif doit pouvoir être mis en oeuvre à un faible coût, pour pouvoir être implémenté chez des personnes bénéficiant de faibles ressources.

### BREVE DESCRIPTION DE L'INVENTION

Conformément à l'invention, il est proposé un dispositif de prédiction et de prévention de l'apparition d'escarres chez une personne comprenant :
- des moyens d'acquisition d'au moins un signal dit « physiologique » représentatif de la formation d'escarres chez une personne, tel que la pression en au moins une zone d'appui du corps de la personne,
- des moyens de transmission dudit signal à un système de traitement,
- un système de traitement apte, à partir des signaux transmis, à déterminer un niveau de risque d'apparition d'escarres,
- des moyens de détermination d'un seuil d'alerte personnalisé,
- des moyens de comparaison dudit niveau de risque avec ledit seuil d'alerte,
- des moyens de déclenchement d'une alerte si le niveau de risque est supérieur audit seuil d'alerte.

Le dispositif comprend également des moyens d'acquisition d'au moins un signal dit « actimétrique » représentatif de l'activité de la personne, par exemple au moins un détecteur de présence installé au domicile de la personne et/ou un système de localisation de la personne à son domicile.

De préférence, ledit dispositif comprend des moyens sans fil pour transmettre le niveau de risque de la personne et/ou une alerte à la personne et/ou à un intervenant désigné au préalable.

Selon un mode préféré de réalisation de l'invention, le dispositif comprend des moyens d'enregistrement des signaux acquis dans une base de données.

En outre, le dispositif comprend avantageusement une interface apte à communiquer à la personne un signal d'alerte, une cartographie de pression dans la zone d'appui et/ou des instructions pour corriger sa posture.

Le dispositif trouve une application particulièrement intéressante dans une utilisation dans un procédé de prévention de l'apparition d'escarres chez une personne, ledit procédé comprenant :
- l'acquisition d'au moins un signal dit « physiologique » représentatif d'un paramètre physiologique de la formation d'escarres, tel que la pression en au moins une zone d'appui,
- la transmission dudit signal à un système de traitement,
- le traitement du(des) signal(aux) transmis au système de traitement pour déterminer un niveau de risque d'apparition d'escarres,
- la détermination d'un seuil d'alerte personnalisé,
- la comparaison du niveau de risque avec ledit seuil d'alerte,
- le déclenchement d'une alerte si le niveau de risque est supérieur au seuil d'alerte.

Ledit procédé comprend l'acquisition d'au moins un signal dit « actimétrique » représentatif de l'activité de la personne, la transmission dudit signal au système de traitement et la prise en compte dudit signal dans la détermination du niveau de risque d'apparition d'escarres.

La détermination du seuil d'alerte comprend les étapes suivantes :
- acquisition de signaux physiologiques représentatifs d'un ou plusieurs paramètres physiologiques de la formation d'escarres, tels que la pression en au moins une zone d'appui du corps de la personne, et/ou
- acquisition de signaux actimétriques représentatifs de l'activité de la personne, et/ou
- acquisition d'informations relatives à l'état de la personne,
- corrélation desdits signaux physiologiques et actimétriques et desdites informations pour en déduire au moins un seuil d'apparition d'escarres spécifique à la personne en fonction de son activité,
- détermination du seuil d'alerte de manière à ce qu'il soit inférieur audit seuil d'apparition d'escarres.

De préférence, lorsqu'une alerte est déclenchée, on communique à la personne une cartographie de pression dans la zone d'appui, et/ou des instructions pour corriger sa posture.

### BREVE DESCRIPTION DES DESSINS

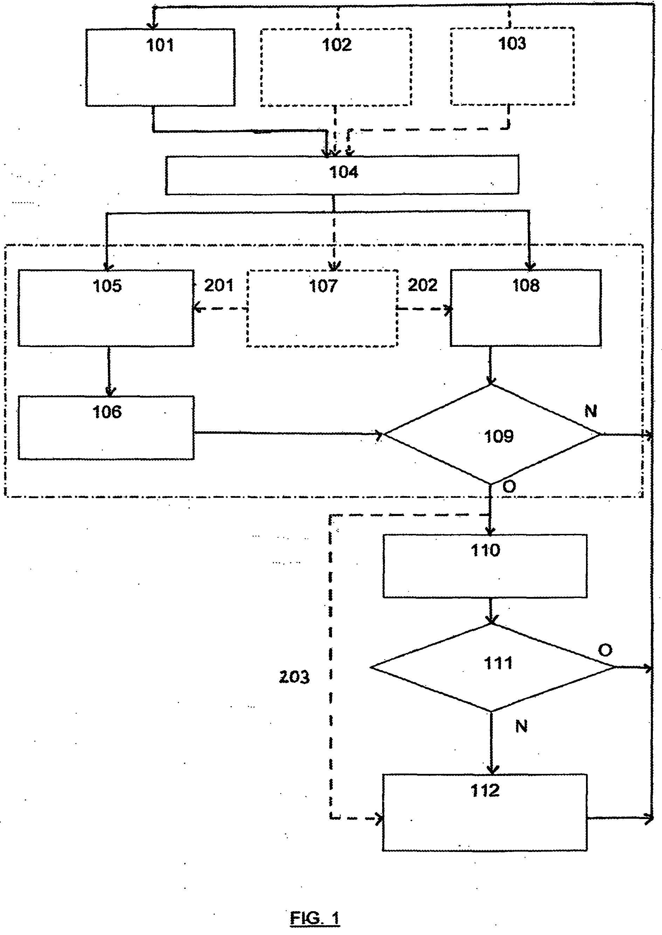

D'autres caractéristiques et avantages de l'invention ressortiront de la description détaillée qui va suivre, en référence à la figure 1 annexée qui présente un logigramme illustrant les étapes du procédé de prévention des escarres conforme à l'invention.

### DESCRIPTION DETAILLEE DE L'INVENTION

### Détermination du seuil d'alerte personnalisé

La détermination d'un seuil d'alerte personnalisé, c'est-à-dire spécifique à la personne, nécessite l'étude de différents paramètres mesurables en continu ou d'informations ponctuelles, liés à la formation des escarres, et leur corrélation avec l'apparition effective d'escarres.

On précise que le seuil d'alerte est une combinaison d'au moins deux paramètres : la pression (et/ou éventuellement d'autres paramètres physiologiques) et le temps.

En effet, il est possible d'atteindre ponctuellement des niveaux de pression élevés sans que ceux-ci ne génèrent d'escarres.

En revanche, l'observation, pendant une durée supérieure à une durée déterminée, d'un niveau de pression élevé est un indice de l'apparition probable d'escarres si la situation n'est pas corrigée.

Parmi les paramètres physiologiques, on peut noter, de manière non limitative, la pression exercée par le corps dans les régions en contact avec le fauteuil ou le lit de la personne : ces régions sont notamment les ischions, trochanter, sacrum ...

Parmi les paramètres morphologiques, on peut mentionner l'indice de masse corporelle (IMC) de la personne.

D'autres paramètres, dits « actimétriques », concernent l'activité de la personne : ainsi une situation immobile pendant une longue durée favorise l'apparition des escarres, notamment si l'attention de la personne est focalisée sur son activité (par exemple, lorsqu'elle regarde la télévision).

D'autres situations à risque peuvent intervenir comme par exemple lorsque la personne se déplace en fauteuil roulant (subissant des chocs répétés) ou bien lorsque la personne bouge sur son fauteuil ou son lit et adopte une position inadéquate.

D'autres informations ponctuelles donnent aussi des indications quant au risque d'apparition des escarres.

Parmi celles-ci, on considère des informations qualitatives, telles que le type de paraplégie (tissus flasques ou non) ou l'apparition de rougeurs dans les zones d'appui : ces rougeurs sont un indicateur du risque de formation d'escarres. L'observation, par le personnel soignant, de la peau de la personne donne donc des informations utiles.

On peut prendre en compte toute autre information sur la personne, telle que ses antécédents médicaux, des résultats d'analyses médicales ou d'évaluation de ses capacités sensori-motrices et cognitives, ou encore des témoignages de la personne ou de son entourage...

Certaines informations sont quantitatives, telles que l'âge, la variation de l'IMC de la personne.

On peut également citer le niveau de gonflage des roues du fauteuil du paraplégique puisqu'un pneu sous gonflé est souvent synonyme de chocs accentués lors de phases de déplacements du fauteuil et donc risque d'apparition d'escarres.

Le processus de détermination du seuil d'alerte comprend une phase d'apprentissage, durant laquelle des mesures sont effectuées et corrélées avec l'apparition réelle d'escarres.

On alimente ainsi une base de données qui permet d'enregistrer l'historique de la formation ou non d'escarres de chaque personne.

Cette base de données permet donc de revenir sur les paramètres associés à la formation d'une escarre. On peut en effet traiter les données collectées en vue d'identifier les facteurs à l'origine de l'apparition des escarres.

De cette base de données peuvent être extraites des informations génériques à l'ensemble des personnes suivies (données inter personnes) mais aussi des informations personnalisées (données intra personne).

A partir des données déjà collectées, il est possible de déterminer au moins un seuil d'apparition d'escarres, c'est-à-dire un ensemble de valeurs des différents paramètres à partir desquelles des escarres se forment. Ce seuil est donc spécifique à la personne.

Par ailleurs, le seuil peut être réactualisé en fonction des nouvelles données collectées. Notamment, le dispositif permet de modifier le modèle si l'on observe un risque d'apparition d'escarres (un indice pouvant être l'apparition d'une rougeur de la peau dans une région concernée).

De manière particulièrement avantageuse, on peut envisager de déterminer selon le même principe plusieurs seuils d'apparition d'escarres, chacun étant lié à une activité particulière de la personne.

Ainsi, une longue immobilisation peut être associée à un seuil bas, tandis qu'une situation où la personne effectue des mouvements pourra être associée à un seuil plus élevé.

Une fois le ou les seuils d'apparition d'escarres déterminés, on définit un ou plusieurs seuils d'alerte, de manière à ce que chaque seuil d'alerte soit inférieur au seuil d'apparition des escarres, pour chaque situation considérée. Le but étant de déclencher une alerte avant que le seuil d'apparition ne soit atteint.

### Mise en oeuvre du procédé

La figure 1 illustre de manière schématique l'agencement des différentes étapes du procédé. Sur ce logigramme, les flèches en pointillés illustrent des étapes optionnelles.

Le procédé comprend l'acquisition, pendant une durée et à une fréquence d'échantillonnage déterminées, d'au moins un signal physiologique représentatif du risque d'apparition d'escarres (étape 101).

Par exemple, il peut s'agir de l'acquisition de signaux relatifs à la pression au niveau des zones d'appui.

De manière particulièrement avantageuse, le procédé comprend en outre l'acquisition de signaux actimétriques par le biais de capteurs embarqués sur la personne ou le fauteuil du paraplégique et de capteurs de présence et d'activité installés dans l'habitat lorsque la personne se trouve à son domicile (étape 102).

Le procédé peut également comprendre l'acquisition d'autres informations, telles que l'IMC de la personne (étape 103).

Par ailleurs, d'autres informations, telles que l'aspect de sa peau peuvent modifier les données d'entrée et de sortie. Ces éléments objectifs participent avantageusement à la modification du modèle permettant de prédire l'apparition des escarres.

L'acquisition peut être réalisée en continu, c'est-à-dire comprendre l'enregistrement de signaux pendant une durée donnée et à une fréquence d'échantillonnage donnée.

En fonction de la personne, l'acquisition peut être effectuée tout au long de la journée ou bien uniquement pendant des plages horaires lors desquelles la personne a une activité à risques.

Ainsi, lorsque des signaux actimétriques sont enregistrés, le procédé peut comprendre une étape de test (non illustrée ici) visant à déterminer si l'activité correspondante est susceptible de favoriser l'apparition d'escarres (par exemple, une situation immobile prolongée) ou non.

L'acquisition des signaux physiologiques n'est alors déclenchée que dans le premier cas.

De manière alternative, le dispositif fonctionne en continu mais avec une fréquence d'acquisition variable selon les moments de la journée et/ou le type d'activité de la personne. Ceci permet de diminuer ta consommation énergétique du dispositif.

D'autres types de signaux font en revanche l'objet d'une acquisition ponctuelle ; il en est ainsi par exemple de l'IMC, de l'état de la peau (indiqué par l'infirmière lors de sa visite)...

Les données acquises à l'étape 101 (et, le cas échéant, aux étapes 102 et/ou 103) sont transmises au système de traitement (étape 104).

La transmission peut être effectuée par tout moyen appropriée, notamment par des moyens sans fil.

Dans le système de traitement ont lieu les étapes suivantes (entourées par le cadre en traits mixtes) :
- une étape 105 de détermination du seuil d'apparition d'escarres spécifique à la personne,
- une étape 106 dérivant de l'étape 105, dans laquelle est déterminé le seuil d'alerte personnalisé.

De manière avantageuse, te traitement comprend en outre une étape 107 d'enregistrement des signaux et autres informations dans une base de données.

Comme exposé plus haut, les données collectées dans cette base peuvent être utilisées pour ajuster le seuil d'apparition des escarres (ce rebouclage est illustré par la flèche 201).

Par ailleurs, le traitement comprend une étape 108 de détermination du niveau de risque de la personne.

Cette détermination est effectuée directement à partir des données transmises au système de traitement en temps réel, mais elle peut également prendre en compte (comme schématisé par la flèche 202) des informations contenues dans la base de données.

Le traitement comprend enfin une étape 109 de test, qui comprend la comparaison du niveau de risque déterminé à l'étape 108 avec le seuil d'alerte déterminé à l'étape 106.

Si le niveau de risque est inférieur au seuil d'alerte (condition N), le système d'acquisition continue d'acquérir au moins un signal physiologique et, le cas échéant, un signal actimétrique et/ou d'autres informations.

Dans le cas où le niveau de risque est supérieur au seuil d'alerte (condition O), une alerte est déclenchée.

Dans le cas où la personne est autonome et apte à prendre en compte les alertes qui lui sont transmises, l'alerte est déclenchée chez la personne (étape 110).

Cette alerte peut être communiquée par tout moyen approprié, par exemple par l'envoi d'un message sur un moyen consultable facilement par la personne, tel qu'un téléphone, notamment un téléphone portable, une montre, un écran installé à proximité de la personne (le cas échéant, incorporé au fauteuil roulant), etc. Bien sûr, ces exemples ne sont en aucun cas limitatifs.

Selon une alternative (représentée par la flèche 203), dans le cas où la personne n'est pas en mesure de prendre connaissance de l'alerte et/ou d'ajuster sa position en fonction de l'alerte, l'alerte est déclenchée chez un intervenant désigné au préalable, par exemple un proche ou un intervenant médical, chargé de se déplacer auprès de la personne pour l'aider à adopter une position plus favorable (étape 112).

De manière avantageuse, le procédé comprend, après le déclenchement de l'alerte chez la personne, une étape 111 de vérification de la prise en compte de l'alerte par la personne.

Ainsi, le dispositif peut comprendre un moyen permettant à la personne d'indiquer qu'elle a bien reçu et compris l'alerte.

Cette vérification peut également être effectuée en surveillant l'évolution du niveau de risque, le maintien d'un niveau de risque élevé pendant une durée déterminée étant un indice d'une mauvaise prise en compte de l'alerte.

Si la personne a pris en compte l'alerte (condition O), l'acquisition d'au moins un signal physiologique et, le cas échéant, d'un signal actimétrique et/ou de toute autre information se poursuit (retour aux étapes 101 et éventuellement 102 et 103).

Ces données seront traitées comme expliqué plus haut et permettront une nouvelle comparaison du niveau de risque avec le seuil d'alerte.

Si la personne n'a pas pris en compte l'alerte (condition N), alors le procédé comprend l'étape 112 de déclenchement de l'alerte chez un intervenant désigné au préalable.

Bien sûr, le logigramme de la figure 1 n'est donné qu'à titre d'illustration du procédé, mais il va de soi que le procédé peut comprendre des étapes supplémentaires ou omettre des étapes facultatives sans pour autant sortir du champ de l'invention.

### Dispositif

Le dispositif comprend tout d'abord des capteurs permettant de mesurer des grandeurs représentatives du risque d'apparition d'escarres.

### Capteurs

Ces capteurs peuvent comprendre notamment : des capteurs de pression (pour mesurer les niveaux de surpression des zones d'appui), des capteurs de type accéléromètre ou gyroscope (pour mesurer l'état postural et l'activité de la personne),...

L'homme du métier connaît ces différents capteurs et leur mode d'utilisation.

Dans le cas d'une personne en fauteuil roulant, les capteurs sont par exemple incorporés au coussin de l'assise.

Outre les capteurs embarqués sur la personne ou en contact avec celle-ci (ou endo-capteurs), le dispositif comprend avantageusement des capteurs permettant de rendre compte de l'environnement de celle-ci.

Ces exo-capteurs peuvent être par exemple :
- des détecteurs de présence installés dans le logement de la personne, en vue de détecter dans quelle pièce celle-ci se trouve et en déduire son activité,
- des dispositifs de localisation embarqués sur le fauteuil roulant,
- des capteurs de gonflage des pneumatiques d'un fauteuil roulant...

Des informations quantitatives peuvent être communiquées directement au système : par exemple, l'IMC de la personne.

Enfin, d'autres informations non quantitatives mais qualitatives peuvent aussi être prises en compte par le système d'acquisition : par exemple, une infirmière peut indiquer l'état de la peau de la personne lors d'un contrôle médical.

### Système d'acquisition

Le système d'acquisition permet d'acquérir les signaux en provenance des différents capteurs mentionnés plus haut.

Selon les cas, l'acquisition des signaux peut être déclenchée à l'initiative de la personne (par exemple, lorsque celle-ci sait qu'elle est dans une situation susceptible de générer des escarres), de manière automatique (par exemple lorsqu'un capteur de présence indique que la personne regarde la télévision) ou encore à l'initiative du médecin avec l'assentiment de la personne concernée (pour vérifier l'état de la personne).

Par ailleurs, le système d'acquisition comprend également, de préférence, une interface permettant à un utilisateur (tel que la personne concernée, le personnel médical) d'entrer directement des données ponctuelles non issues des capteurs (telles que l'IMC, l'état de la peau...).

La liaison entre les capteurs et le système d'acquisition est réalisée par des moyens connus en eux-mêmes, et qui ne seront donc pas décrits de manière détaillée.

De manière avantageuse, la liaison est sans fil et utilise toute technologie adéquate..

### Système de traitement

Le système de traitement prend en compte l'ensemble des données issues des capteurs et, le cas échéant, des informations renseignées directement par un utilisateur, et en déduit un niveau de risque d'apparition d'escarres.

Le système de traitement compare ce niveau de risque au seuil d'alerte personnalisé décrit plus haut, et déclenche une alerte si le niveau de risque est supérieur au seuil d'alerte.

Dans un autre mode de réalisation simplifié, le système de traitement est autonome, c'est-à-dire qu'il est apte, à partir des informations en provenance du système d'acquisition, à déterminer le niveau de risque et à le comparer avec le seuil d'alerte.

Ce mode de réalisation correspond à un système de traitement local (par rapport à la personne), par exemple embarqué sur le fauteuil de la personne.

Dans un mode de réalisation plus complet, le système de traitement comprend la base de données évoquée plus haut. La base de données nécessitant des moyens (notamment une mémoire) plus puissants, le système de traitement est éloigné de la personne et communique avec le système d'acquisition et le système d'alerte installés chez la personne par des moyens appropriés. Dans ce cas, le système de traitement est de préférence commun à plusieurs personnes, le système d'acquisition et le système d'alerte étant quant à eux installés localement, c'est-à-dire chez la personne.

### Système d'alerte

L'alerte peut être transmise à la personne et/ou au corps médical par tout moyen adapté à la prise en compte par son destinataire.

Ainsi, dans le cas de personnes para ou tétraplégiques, différents dispositifs de stimulation ont déjà été décrits. Parmi ceux-ci, on peut citer le stimulateur endo-buccal mentionné dans le document WO 2006/008406.

De manière avantageuse, l'alerte comprend l'indication à la personne des régions à risque.

Selon les cas, la personne définit elle-même le changement de posture à adopter pour réduire le risque, par exemple à partir d'une cartographie des pressions dans les zones d'appui, ou bien le système d'alerte lui transmet des instructions plus précises quant à la posture à adopter.

L'alerte peut également être transmise à une interface (par exemple un écran) sous la forme d'un message écrit lisible et compréhensible directement par l'utilisateur (qu'il s'agisse de la personne ou du corps médical).

Comme évoqué plus haut, les moyens de liaison entre le système de traitement et le système d'alerte peuvent comprendre tous les moyens existants, de préférence des moyens sans fil.

En vue de minimiser le coût du dispositif, le système d'alerte est avantageusement incorporé à un moyen existant, tel qu'un téléphone portable, une montre, etc. L'installation du système d'alerte ne nécessite alors que la programmation de la fonctionnalité souhaitée.

L'interface de lecture de l'alerte peut prendre différentes formes, en fonction du destinataire.

L'interface peut être un écran intégré au fauteuil roulant de la personne, ou bien une montre bracelet munie d'un vibreur signalant à la personne (ou au corps médical) qu'il doit prendre connaissance d'un message. Dans ce dernier cas, la montre comporte avantageusement un écran pour l'affichage d'un message.

De préférence, on effectue une acquisition quotidienne des données physiologiques et actimétriques qui sont transmises à la base de données pour une prise en compte d'éventuelles modifications.

Par ailleurs, toutes ces informations peuvent alimenter une base de données comportementales, posturales et physiologiques associées à la personne concernée, afin d'affiner en continu la détermination des seuils d'alerte.

De manière particulièrement avantageuse, le dispositif comprend également des moyens pour envoyer un message, par exemple à la fin de la journée, pour indiquer le niveau de risque, si l'alerte n'a pas été déclenchée au cours de ta journée. Ceci permet de rassurer la personne ou son entourage sur l'absence de risque à court terme.

### Exemples de situations de vie

### Cas de la personne devant la télévision

Il a été constaté que le fait de regarder la télévision a tendance à favoriser l'apparition des escarres, dans la mesure où toute l'attention de la personne est focalisée sur l'émission et non sur la nécessité de modifier régulièrement sa posture.

Il est donc particulièrement important de surveiller l'état de la personne dans cette situation.

Pour résoudre ce problème, on propose d'équiper le domicile de la personne d'un détecteur de présence dans la pièce où se trouve la télévision, voire d'un capteur du niveau sonore dans la pièce, afin de déterminer lorsque la personne regarde une émission.

Lorsque cette situation est détectée, l'acquisition des signaux physiologiques se déclenche, et donne lieu à l'émission d'une alerte si le seuil d'alerte est dépassé.

### Cas de la personne se déplaçant en fauteuil roulant

Une autre situation à risques est celle de la personne en fauteuil roulant, qui se déplace sur un sol non lisse (par exemple dans une rue, avec des franchissements de trottoirs).

En effet, lorsque la personne subit des chocs lors du roulage, sa posture peut se modifier légèrement et favoriser l'apparition d'escarres.

Il peut donc être utile de surveiller l'état de la personne pendant et après un tel trajet.

Par ailleurs, le gonflage des pneus du fauteuil a une incidence sur la transmission des chocs ; par conséquent, il sera avantageux d'enregistrer le niveau de pression des pneus et de déclencher une alerte si l'on détecte qu'un pneu est dégonflé.

### Cas de la télésurveillance de plusieurs personnes

L'invention trouve avantageusement une application dans un centre de surveillance d'une pluralité de personnes. Ce centre de surveillance peut être situé à distance pour surveiller des personnes maintenues chacune à leur domicile, ou bien situé sur un plateau, médicalisé ou non, sur lequel sont installées plusieurs personnes..

Pour faciliter la surveillance par le personnel du centre de surveillance, chacune des personnes est équipée des capteurs appropriés tels que décrits plus haut.

Un système d'acquisition enregistre les signaux en provenance de chacune des personnes.

Un système de traitement centralisé traite les signaux pour en déduire le niveau de risque de chaque personne et le comparer au seuil d'alerte spécifique à chaque personne.

Ces informations sont transmises à un système d'information se présentant par exemple sous la forme d'un écran permettant au personnel de surveillance de visualiser simultanément la situation de chaque personne.

Lorsqu'une alerte est déclenchée, elle peut se traduire par un signal visuel et/ou sonore.

De manière particulièrement avantageuse, ce système peut être complété par un dispositif permettant d'alerter le personnel de surveillance même lorsqu'il n'est pas devant l'écran.

Ce dispositif comprend par exemple une montre bracelet munie d'un vibreur tactile.

Le personnel du centre dispose d'un écran sur lequel s'affichent les informations relatives à chaque personne, celles-ci étant par exemple communiquées par une technologie sans fil.

## Revendications

1. Dispositif de prévention de l'apparition d'escarres chez une personne comprenant :
- des moyens d'acquisition d'au moins un signal dit « physiologique » représentatif de la formation d'escarres chez une personne, tel que la pression en au moins une zone d'appui du corps de la personne,
- des moyens de transmission dudit signal à un système de traitement,
- un système de traitement apte, à partir des signaux transmis, à déterminer un niveau de risque d'apparition d'escarres,
- des moyens de détermination d'un seuil d'alerte personnalisé,
- des moyens de comparaison dudit niveau de risque déterminé avec ledit seuil d'alerte personnalisé, et
- des moyens de déclenchement d'une alerte si ledit niveau de risque est supérieur audit seuil d'alerte,
**caractérisé en ce qu'**il comprend en outre :
- des moyens d'acquisition d'au moins un signal dit « actimétrique » représentatif de l'activité de la personne, pour sa transmission au système de traitement.

2. Dispositif selon la revendication 1, **caractérisé en ce que** lesdits moyens d'acquisition de signaux actimétriques comprennent au moins un détecteur de présence installé au domicile de la personne et/ou un système de localisation de la personne à son domicile.

3. Dispositif selon l'une des revendications 1 ou 2, **caractérisé en ce qu'**il comprend des moyens sans fil pour transmettre le niveau de risque de la personne à la personne et/ou à un intervenant désigné au préalable.

4. Dispositif selon l'une des revendications 1 à 3, **caractérisé en ce qu'**il comprend des moyens d'enregistrement des signaux acquis dans une base de données.

5. Dispositif selon l'une des revendications 1 à 4, **caractérisé en ce qu'**il comprend une interface apte à communiquer à la personne un signal d'alerte, une cartographie de pression dans la zone d'appui et/ou des instructions pour corriger sa posture.

6. Procédé pour déclencher une alerte dans la prévention de l'apparition d'escarres chez une personne, ledit procédé comprenant :
- l'acquisition d'au moins un signal dit « physiologique » représentatif d'un paramètre physiologique de la formation d'escarres, tel que la pression en au moins une zone d'appui,
- l'acquisition d'au moins un signal dit « actimétrique » représentatif de l'activité de la personne,
- la transmission desdits signaux physiologique et actimétrique à un système de traitement,
- le traitement des signaux transmis au système de traitement pour déterminer un niveau de risque d'apparition d'escarres,
- la détermination d'un seuil d'alerte personnalisé,
- la comparaison du niveau de risque avec ledit seuil d'alerte,
- le déclenchement d'une alerte si le niveau de risque est supérieur au seuil d'alerte.

7. Procédé selon la revendication 6, **caractérisé en ce que** la détermination du seuil d'alerte comprend les étapes suivantes :
- acquisition de signaux physiologiques représentatifs d'un ou plusieurs paramètres physiologiques de la formation d'escarres, tels que la pression en au moins une zone d'appui du corps de la personne, et/ou
- acquisition de signaux actimétriques représentatifs de l'activité de la personne, et/ou
- acquisition d'informations relatives à l'état de la personne,
- corrélation desdits signaux physiologiques et actimétriques et desdites informations pour en déduire au moins un seuil d'apparition d'escarres spécifique à la personne en fonction de son activité,
- détermination du seuil d'alerte de manière à ce qu'il soit inférieur audit seuil d'apparition d'escarres.

8. Procédé selon l'une des revendications 6 ou 7, **caractérisé en ce qu'**il comprend, lorsqu'une alerte est déclenchée, l'étape consistant à communiquer à la personne une cartographie de pression dans la zone d'appui, et/ou des instructions pour corriger sa posture.

## Patentansprüche

1. Vorrichtung zur Vorbeugung des Auftretens von Schorf bei einer Person, umfassend:
- Empfangsmittel mindestens eines für die Ausbildung von Schorf bei einer Person repräsentativen "physiologischen" Signals wie der Druck in mindestens einer Stützzone des Körpers der Person,
- Übertragungsmittel des Signals an ein Verarbeitungssystem,
- ein Verarbeitungssystem, das imstande ist, ausgehend von den übertragenen Signalen ein Risikoniveau für das Auftreten von Schorf zu bestimmen,
- Bestimmungsmittel einer persönlichen Warnstufe,
- Vergleichsmittel des bestimmten Risikoniveaus mit der persönlichen Warnstufe, und
- Auslösemittel einer Warnung, wenn das Risikoniveau höher als die Warnstufe ist,
**dadurch gekennzeichnet, dass** sie ferner umfasst:
- Empfangsmittel mindestens eines "aktigraphischen" Signals, das für die Aktivität der Person repräsentativ ist, für ihre Übertragung an das Verarbeitungssystem.

2. Vorrichtung nach Anspruch 1, **dadurch gekennzeichnet, dass** die Empfangsmittel aktigraphischer Signale mindestens einen in der Wohnung der Person installierten Anwesenheitsdetektor und/oder ein Lokalisierungssystem der Person in ihrer Wohnung umfassen.

3. Vorrichtung nach einem der Ansprüche 1 oder 2, **dadurch gekennzeichnet, dass** sie drahtlose Mittel umfasst, um das Risikoniveau der Person an die Person und/oder an einem zuvor benannten Beteiligten zu übertragen.

4. Vorrichtung nach einem der Ansprüche 1 bis 3, **dadurch gekennzeichnet, dass** sie Speichermittel der empfangenen Signale in einer Datenbank umfasst.

5. Vorrichtung nach einem der Ansprüche 1 bis 4, **dadurch gekennzeichnet, dass** sie eine Schnittstelle umfasst, die imstande ist, an die Person ein Warnsignal, eine Kartographie des Drucks in der Stützzone und/oder Anweisungen zur Korrektur ihrer Haltung zu übertragen.

6. Verfahren zum Auslösen einer Warnung zur Vorbeugung des Auftretens von Schorf bei einer Person, wobei das Verfahren umfasst:
- das Empfangen mindestens eines "physiologischen" Signals, das für einen physiologischen Parameter der Ausbildung von Schorf repräsentativ ist, wie der Druck in mindestens einer Stützzone,
- das Empfangen mindestens eines "aktigraphischen" Signals, das für die Aktivität der Person repräsentativ ist,
- die Übertragung des physiologischen und aktigraphischen Signals an ein Verarbeitungssystem,
- die Verarbeitung der an das Verarbeitungssystem übertragenen Signale zwecks Bestimmung eines Risikoniveaus für das Auftreten von Schorf,
- das Bestimmen einer persönlichen Warnstufe,
- das Vergleichen des Risikoniveaus mit der Warnstufe,
- das Auslösen einer Warnung, wenn das Risikoniveau höher als die Warnstufe ist.

7. Verfahren nach Anspruch 6, **dadurch gekennzeichnet, dass** die Bestimmung der Warnstufe die folgenden Schritte umfasst:
- Empfangen physiologischer Signale, die für einen oder mehrere physiologische Parameter der Ausbildung von Schorf repräsentativ sind, wie der Druck in mindestens einer Stützzone des Körpers der Person, und/oder,
- Empfangen aktigraphischer Signale, die für die Aktivität der Person repräsentativ sind, und/oder
- Empfangen von Informationen über den Zustand der Person,
- Korrelieren der physiologischen und aktigraphischen Signale und der Informationen, um daraus mindestens eine Stufe für das Auftreten von Schorf abzuleiten, die für die Person in Abhängigkeit von ihrer Aktivität spezifisch ist,
- Bestimmen der Warnstufe derart, dass diese unter der Stufe für das Auftreten von Schorf liegt.

8. Verfahren nach einem der Ansprüche 6 oder 7, **dadurch gekennzeichnet, dass** es, wenn ein Alarm ausgelöst wird, den Schritt umfasst, der darin besteht, an die Person eine Kartographie des Drucks in der Stützzone und/oder Anweisungen zur Korrektur ihrer Haltung zu übertragen.

## Claims

1. A device for preventing the occurrence of pressure sores in a person comprising:
- means for acquiring at least one so-called "physiological" signal representative of the formation of pressure sores in a person, such as the pressure in at least one supporting area of the body of the person,
- means for transmitting said signal to a processing system,
- a processing system capable, from the transmitted signals, of determining a level of risk of occurrence of pressure sores,
- means for determining a customized alert threshold,
- means for comparing said determined risk level with said customized alert threshold, and
- means for triggering an alert if said risk level is greater than said alert threshold,
**characterized in that** it further comprises:
- means for acquiring at least one so-called "actimetric" signal representative of the activity of the person, for transmitting it to the processing system.

2. The device according to claim 1, **characterized in that** said means for acquiring actimetric signals comprise at least one presence detector installed in the home of the person and/or a system for localizing the person in his/her home.

3. The device according to one of claims 1 or 2, **characterized in that** it comprises wireless means for transmitting the risk level of the person to the person and/or a previously designated intervenor.

4. The device according to one of claims 1 to 3, **characterized in that** it comprises means for recording the acquired signals in a database.

5. The device according to one of claims 1 to 4, **characterized in that** it comprises an interface capable of transmitting to the person, an alert signal, a pressure mapping in the supporting area and/or instructions for correcting his/her posture.

6. A method for triggering an alert in the prevention of occurrence of pressure sores in a person, said method comprising:
- acquiring at least one so-called "physiological" signal representative of a physiological parameter of the formation of pressure sores in a person, such as the pressure in at least one supporting area,
- acquiring at least one so-called "actimetric" signal representative of the activity of the person,
- transmitting said physiological and actimetric signals to a processing system,
- processing the signals transmitted to the processing system for determining a level of risk of occurrence of pressure sores,
- determining a customized alert threshold,
- comparing the risk level with said alert threshold,
- triggering an alert if the risk level is greater than the alert threshold.

7. The method according to claim 6, **characterized in that** the determination of the alert threshold comprises the following steps:
- acquiring physiological signals representative of one or several physiological parameters of the formation of pressure sores, such as the pressure in at least one supporting area of the body of the person, and/or
- acquiring actimetric signals representative of the activity of the person, and/or
- acquiring information relating to the condition of the person,
- correlating said physiological and actimetric signals and said information in order to infer therefrom at least one pressure sore occurrence threshold specific to the person depending on his/her activity,
- determining the alert threshold so that it is lower than said pressure sore occurrence threshold.

8. The method according to one of claims 6 or 7, **characterized in that** it comprises, when an alert is triggered, the step consisting of transmitting to the person a pressure mapping in the supporting area, and/or instructions for correcting his/her posture.
